# EUROPEAN PATENT SPECIFICATION

(11) **EP 1 579 829 B1**
(45) Date of publication and mention of the grant of the patent: **13.09.2006**
(21) Application number: 05075680.8
(22) Date of filing: 18.03.2005
(51) Int. Cl.: A61F 5/01

(54) **Articulated rod with assisted regulation for an orthopedic brace**
Gelenkstange mit unterstützter Einstellung für eine Gelenkorthese
Tige articulée avec régularisation assistée pour une attelle orthopédique

(30) Priority: 24.03.2004 IT MI20040574
(43) Date of publication of application: 28.09.2005
(73) Proprietor: Orthoschärer & Co. di Paolo Rossi & Co., 6362 Stansstad (NW) (CH)
(72) Inventor: Rossi Paolo, 6362 Stansstad (CH); Caporello Paolo, 31044 Montebelluna (IT)
(74) Representative: Coppo, Alessandro

(56) References cited:
- US-A- 6 039 709
- US-A1- 2003 199 798
- US-A1- 2004 015 112
- US-B1- 6 203 511

## Description

The present invention relates to an articulated rod with assisted regulation for an orthopedic brace.

Orthopedic braces, such as, for example, hip braces or shoulder braces are surgical prostheses prescribed for people suffering from chronic dislocation problems.

Hip braces are used for patients whose femur head tends to become easily dislocated from the acetabulum, i.e. the hip socket. Dislocation of the hip is a pathology which frequently arises in patients carrying hip prostheses, thus running the risk of having to undergo a further surgical intervention. The hip prosthesis is implanted in a high number of elderly people, mainly women, following a fracture of the head of the femur or serious phenomena due to arthritis.

Hip braces consisting of a first element suitable for being worn on the pelvis and commonly called "pelvis- grips" connected by means of an articulated rod to a second element suitable for being fitted on a thigh, called "thigh-grip", have been present on the market for some time. The articulated rod limits the flexo-extension movement of the leg to an extent which is not dangerous for the patient.

Articulated rods generally consist of a first element to be connected to the pelvis-grip and a second element to be connected to the thigh-grip, which are hinged to each other.

European Patent EP 1068845 discloses for example the production of articulated rods by means of a hinge device consisting of two superimposed plates joined in the rotation centre, each of which is integral with an element of the articulated rod. The lower plate has a series of equidistant holes on its perimeter, in which elements, pins or screws, are inserted to limit the maximum relative rotation extent between the plates.

Said pins or screws, which protrude with respect to the lower plate, form a stop or wedge surface for the upper plate whose rotation with respect to the lower plate is therefore limited within a preestablished range.

In order to regulate the maximum rotation angle between the portions of articulated rods, the medical staff must consequently remove the screws or pins and position them in the new desired position. This operation not only requires the use of screw-drivers or other specific tools, but is also laborious and also creates the risk of losing or damaging the screws or pins as well as the housing containing them, for example consisting of threaded holes.

Another example of an articulated rod with an adjustable range of rotation has been disclosed in US Patent US 6,203,511.

An objective of the present invention is to provide an articulated rod with assisted regulation for an orthopedic brace which can be rapidly and accurately regulated.

A further objective of the present invention is to provide an articulated rod which is particularly simple and functional, with reduced costs.

These objectives according to the present invention are achieved by providing an articulated rod with assisted regulation for an orthopedic brace as indicated in claim 1.

Further characteristics are envisaged in the dependent claims.

The characteristics and advantages of an articulated rod with assisted regulation for an orthopedic brace according to the present invention will appear more evident from the following illustrative but non-limiting description, referring to the enclosed schematic drawings, in which:
figure 1 shows an articulated rod with assisted regulation for an orthopedic brace according to the invention assembled on a hip brace;
figure 2 is a perspective view of an articulated rod with assisted regulation for an orthopedic brace in a straight position;
figure 3 is an enlarged and partially cross-sectioned perspective view of the rear side of the articulated rod according to the invention in an angled position;
figures 4 to 6 show a plan view of a partially cross-sectioned enlarged detail of the hinge device of the articulated rod according to the present invention, in various operative positions.

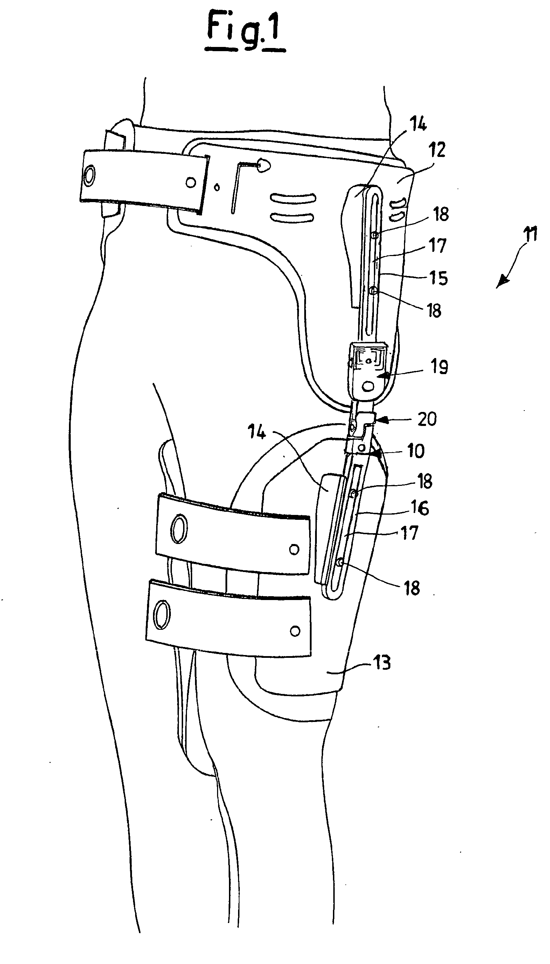

With reference to the figures, these illustrate an articulated rod with assisted regulation 10 for an orthopedic brace, which in the example shown in figure 1 is a hip brace indicated as a whole with 11. The articulated rod 10 according to the present invention could also be used in other orthopedic braces, such as shoulder braces, not shown.

The hip brace 11, shown for illustrative and non-limiting purposes, comprises a pelvis-grip 12 which is connected by means of the articulated rod 10 to a thigh-grip 13.

The pelvis-grip 12 and the thigh-grip 13 both have suitable housings 14 for a first element, or first portion of the rod, 15 and a second element, or second portion of the rod, 16 respectively, of the articulated rod 10.

Figure 1, for example, shows grooves or slits 17 in which screws 18 are inserted for fixing the rod 10 to the pelvis-grip 12 and thigh-grip 13 and for regulating the brace 11 on the basis of the patient's height.

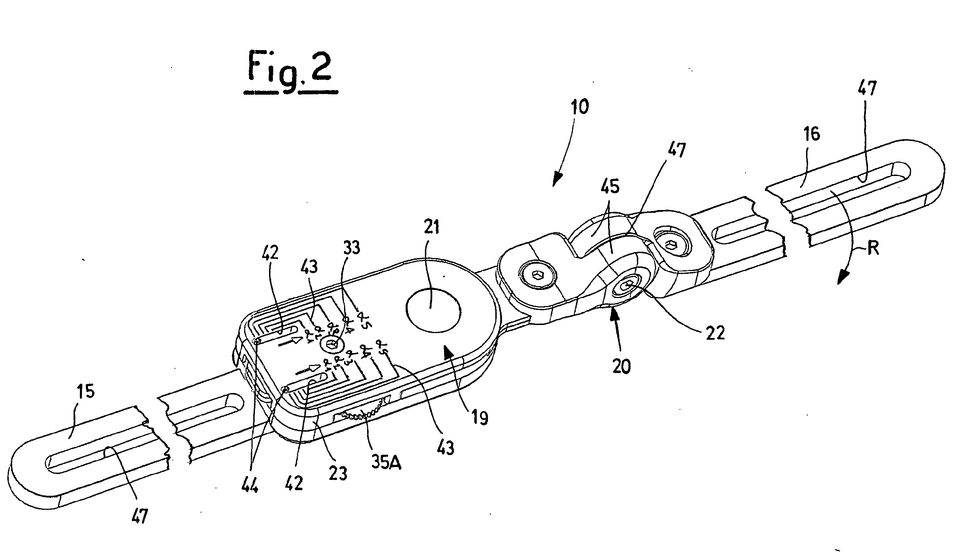

The first portion 15 and the second portion 16, which form the rod 10, are reciprocally joined by means of an articulated portion, shown in figures 2 to 6, comprising a hinge device 19 for a rotational movement around a first rotation axis 21 in a first flexo-extension plane, and an articulated joint 20 for an angular regulation around a second rotation axis 22 in a second abduction-adduction plane, orthogonal with each other.

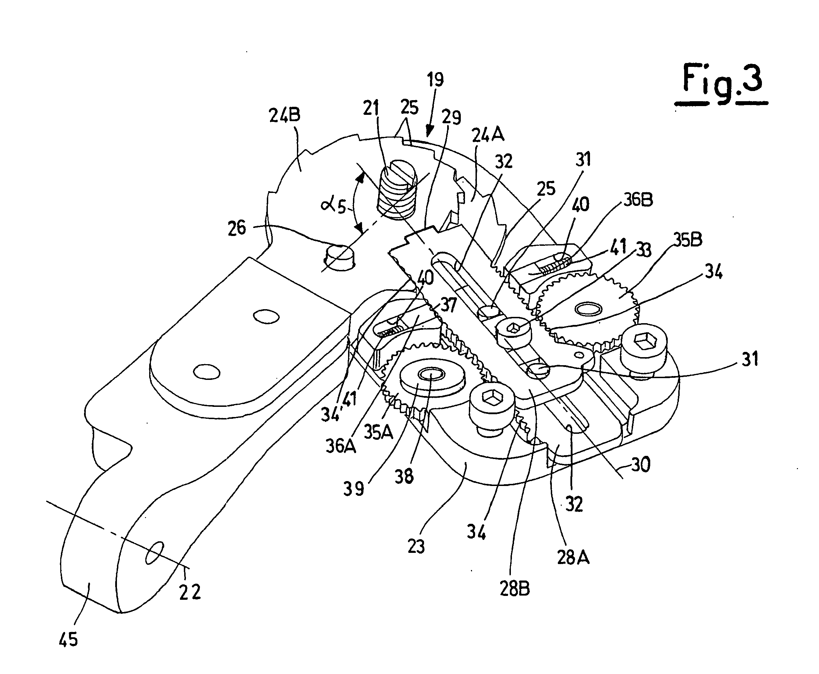

The hinge device 19 comprises a shell 23, which in figures 3 to 6 is illustrated partially removed, and in the example it is firmly applied to the first portion of the rod 15 integral with the pelvis-grip 12. The hinge device 19 also comprises a pair of disks 24 firmly applied to the second portion of the rod 16, which in the example is integral with the thigh-grip 13.

The disks 24 each have a perimeter with an increasing diameter equipped with a series of teeth, or steps 25 and are arranged inside the shell 23, superimposed so that the toothed profiles of an upper disk 24A and a lower disk 24B do not coincide but are opposite each other.

Each disk has a number of teeth 25 equal to the angular positions which can be positioned between the two rod portions 15 and 16. In the example shown, the disks 24 are each equipped with nine teeth 25 and the second rod portion 16, in addition to being in an aligned position with the first rod portion 15 of figure 2, can in fact be situated on each side in four different angular positions, rotated by an angle α equal for example to 20°, 45°, 75° and 110°.

The upper disk 24A and the lower disk 24B, made integral with each other by means of a peg 26 inserted in a pair of aligned holes, can be integrally rotated together around a pin which defines the rotation axis 21 of the hinge device 19.

The shell 23 also comprises a pair of slides 28, an upper slide 28A and a lower slide 28B, superimposed and coplanar with the pair of disks 24. Each of the slides 28A and 28B has a tooth 29, or protruding element, at one end, suitable for corresponding to one of the perimetric teeth 25 of the disks 24A and 24B, allowing a different rotation of the pair of disks 24 depending on the position of the pair of slides 28.

The slides 28A and 28B are in fact each positioned in correspondence with regulation means which cause them to move towards or away from the disks 24A and 24B, by means of a translation of each of the slides 28 along a translation axis 30 orthogonal with the rotation axis 21 of the hinge device 19.

The translation of the slides 28 can be guided, for example, by two pegs 31 integral with the shell 23 and inserted in a central slit 32 of the slides 28, which is parallel to the translation axis 30. The slits 32 also house a blocking screw 33, which, when externally tightened, firmly fixes the angular position between the rod portions 15 and 16.

According to a preferred embodiment of the articulated rod 10, each of the slides 28 has toothing, or a series of crests and cavities, on opposite sides and parallel to the translation axis 30, to be coupled with the regulation means which intervene on opposite sides of the slides 28A and 28B.

A first toothing 34 of the slides 28A and 28B corresponds with toothed wheels 35A and 35B respectively, i.e. a first regulation device. A second toothing 34', different from the first toothing 34 and having a greater pitch and corresponding to an angular pitch, is coupled with further regulation devices, consisting of cursors 36A and 36B, respectively.

The two wheels 35A and 35B are each positioned on a fixed pin 38 on opposite sides with respect to the slides 28A and 28B, and are also coplanar with the same. On the fixed pin 38, there is also a ring, or washer 39, made of plastic antifriction material, in contact with the regulation device and coplanar with one of the slides 28.

Each cursor 36A and 36B is made of an antifriction material and has a toothed portion 37 complementary to the toothing 34'. As illustrated in figure 3, the cursors 36A and 36B are housed in the shell 23 in houses 40 each together with an elastic element 41, which keeps the toothed portion 37 of the cursor in correspondence with the toothing 34'.

The translation movement, for example, of the upper slide 28A causes the cursor 36A to "jump" onto the toothing 34' of the slide 28A from one tooth to the next. The cursor 36A is in fact forced to become disengaged from the slide 28A by compressing the elastic element 41 which, when charged, brings the cursor 36A back into contact with the slide 28A immediately after the advancing of a tooth of the toothing 34'.

The slide 28A can therefore take on discreet positions along the translation axis 30, determined by the pitch of the toothing 34', corresponding with a series of discreet angular positions of the two portions 15 and 16 of the articulated rod 10, object of the present invention.

The desired angular position is established by the user by intervening on the two regulation wheels 35A and 35B, which are housed in the shell 23, partially protruding outwards and which interlock direct with the toothing 34 of the slides 28A and 28B.

The shell 23 is also equipped with at least one window 42, or opening, for the reading on a graded scale 43 of the position of the slides and consequently of the angular position established between the rod portions. In particular, in each of two windows 42 there is a moveable indicator 44 integral with each slide 28.

Figure 2 indicates, for purely illustrative purposes, the angles α₁, α₂, α₃, α₄, α₅ which could be, for example, 0°, 20°, 45°, 75° and 110°.

In the preferred embodiment of the articulated rod 10 shown for illustrative purposes, the articulated joint 20, which forms an angular rotation in the abduction-adduction plane around the second axis 22, is situated between the hinge device 19 and the second rod portion 16 connected to the thigh-grip 13. The articulated joint 20 comprises two disk portions 45 respectively connected to the pair of disks 24 and to an end of the second rod portion 16. The disk portions 45 have a central pass-through hole for the insertion of a regulation screw which identifies the rotation axis 22. On flat facing surfaces, the disk portions 45 are equipped with friction means 47, such as complementary toothing.

The articulated joint 20 is shown and described for illustrative purposes only and may also be absent or a different articulated joint known to experts in the field, suitable for allowing even more complex movements.

The articulated rod with assisted regulation, object of the present invention, consists, in a preferred embodiment, of a light aluminum alloy structure.

An exception is represented by the toothed elements which are made of steel of other low-wear material.

In addition, sliding parts are preferably made of or lined with an antifriction plastic materials.

The use and functioning of an articulated rod with assisted regulation 10 for a hip brace according to the invention is substantially as follows.

Once the brace 11 has been inserted and the articulated rod 10 fixed thereto, the orthopedic technician or doctor, regulates the hinge device 19 to allow the two rod portions 15 and 16 to assume the relative positions enabling a maximum movement range which is compatible with the patient's conditions. The two rod portions 15 and 16 can be optionally blocked in a certain fixed position, aligned with each other or in a position in which the second rod portion 16 is rotated by an angle α with respect to the position with the rods 15 and 16 aligned (figure 2).

The articulated rod with assisted regulation 10, object of the present invention, is suitable for being applied both to the left leg and right leg of the brace 11 as the second rod portion 16, with respect to the starting position shown in figure 2, in which it is aligned with the first rod portion 15, can have an angle α ranging from about 0° to 110° on both sides.

In the production of the rod 10 shown for illustrative purposes, four angular regulations can be established on each side, in particular with the angle α₂ equal to 20°, α₃ equal to 45°, α₄ equal to 75°, α₅ equal to 110°.

In order to use the articulated rod 10 on the left leg of the brace 11, as shown in figure 1, and allow, for example, a movement of the second rod portion 16 equal to an angle α ranging from 0° to 45°, the doctor will act on the regulation wheel 35A situated on the side towards which the rotation is directed, until the indicator 44 indicates the desired angle on the graded scale, for example α₃ equal to 45°. The other wheel 35B is moved to bring the indicator 44 to the position α₁ equal to 0°.

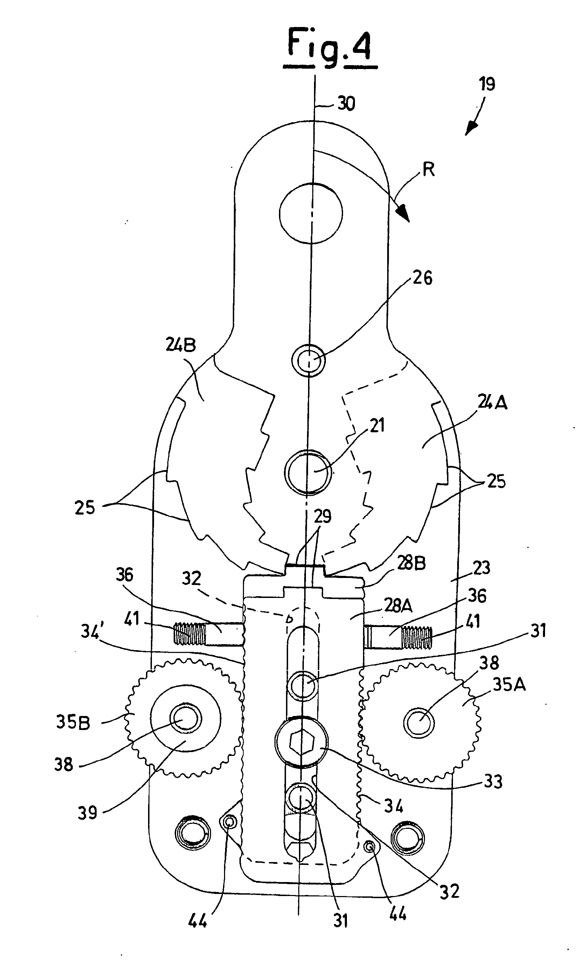

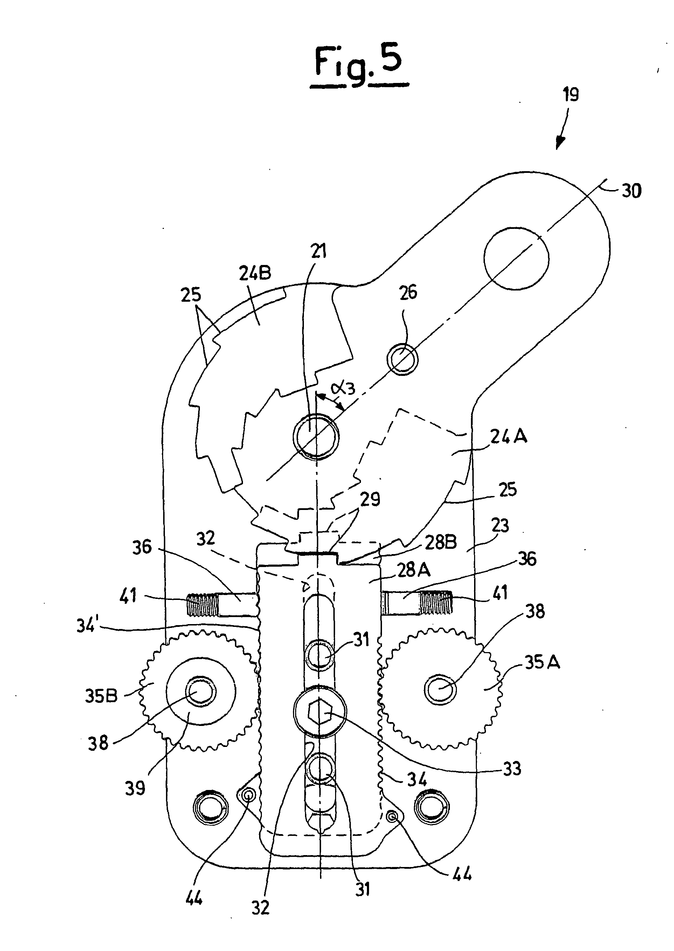

In this position, shown in the enlarged detail of figure 4, the engaging element 29, situated at the end of the upper slide 28A, is not yet in contact with the toothed perimeter 25 of the upper disk 24A. The rod portion 16 can therefore be rotated according to the arrow R until a tooth 25, of the toothed profile of the upper disk 24A, is wedged with the protruding element 29 of the upper slide 28A, as shown in figure 5. In particular, for the regulation with the angle α₃ equal to 45°, the engagement with the slide 28A will be effected with the third tooth of the upper disk 24A, counting from the greater diameter and therefore in a clockwise direction.

The engaging element 29 of the lower slide 28B is, on the other hand, in contact with a tooth 25 of the lower disk 24B, in particular with the central tooth 25, when the rod portions 15 and 16 are aligned with each other, thus preventing the rotation in an anticlockwise direction, and therefore opposite to the direction of the arrow R, of the second rod portion 16.

An angular movement from 0° to 45° is thus possible for the articulated rod 10 of figure 5.

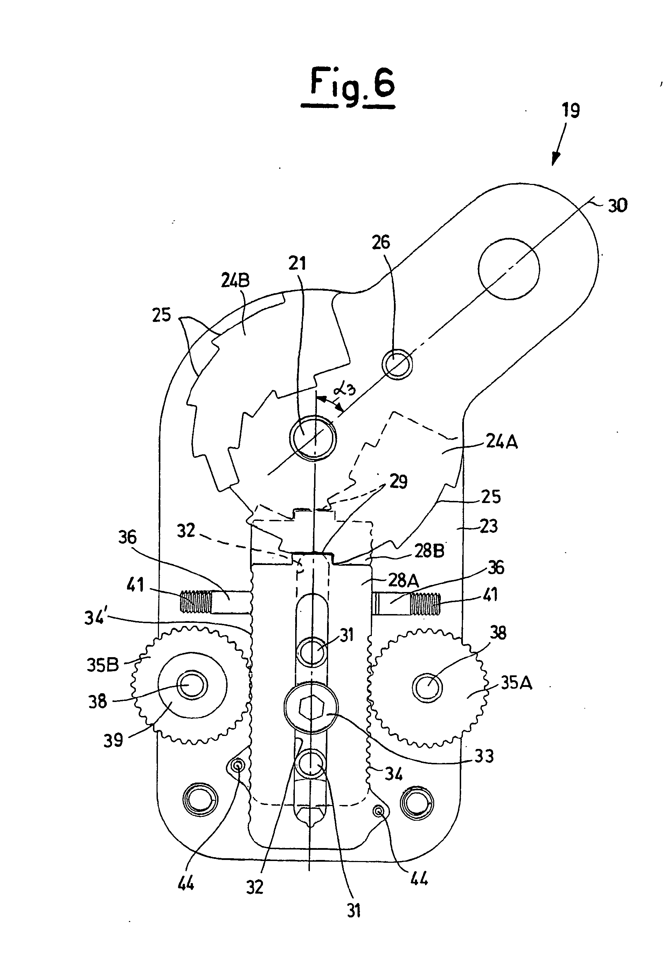

In order to fix the position of the rod portion 16, for example in the position with an angle α₃ equal to 45°, the wheel 35B, corresponding to the indicator 44 which is situated in an angle position α₁ equal to 0°, is moved to bring the indicator 44 to the run-end for blockage. This corresponds to engaging the respective slide 28B against the toothed profile 25 of the relative lower disk 24B (figure 6) against the seventh tooth 25 counting from the greater diameter and therefore in an anticlockwise direction.

After regulating the position of the slides 28 by intervening on the wheels 35A and 35B, the blockage screw 33 is tightened to ensure that the rod 10 under load is firmly gripped.

Furthermore, the regulation of the articulated joint 20 allows the rod 10 to be adapted to the patient's physical conformation. A loosening of the screw, in fact, causes the disengagement of the flat facing surfaces of the disk portions 45, equipped with teeth 47, thus allowing the relative rotation. Once the abduction angle to be enforced on the outstretched leg, has been reached, the screw can be tightened.

The articulated rod with assisted regulation for a brace, in particular for a hip brace, object of the present invention, has the advantage of being simple to apply to the brace and can be easily and accurately regulated.

The regulation of the angular position between the two rod portions can in fact be effected by the medical staff, by acting manually on the regulation wheels without requiring the use of screwdrivers or other tools for removing or moving the screws and other regulation elements.

The regulation can be advantageously effected in a rapid and accurate manner without running the risk of losing screws or other elements to be removed.

Numerous modifications and variations can be applied to the articulated rod with assisted regulation for a hip brace thus conceived, all included within the scope of the invention; furthermore all the details can be substituted by technically equivalent elements. In practice, the materials used, as also the dimensions, can vary according to technical demands.

## Claims

1. An articulated rod with assisted regulation for an orthopedic brace comprising two portions of rod (15, 16) joined by at least one hinge device (19) for a rotational movement around a first rotation axis (21) of the brace, said rod portions (15, 16) being firmly connected to a first and second element of an orthopedic brace (12, 13), **characterized in that** said hinge device (19) comprises a shell (23) and a pair of disks (24) firmly applied to said portions of rod (15, 16), wherein each of the disks (24A, 24B) has a perimeter with an increasing diameter equipped with a series of teeth (25), or steps, said disks (24) being superimposed in said shell (23) with opposite toothed profiles (25), and which can be integrally rotated together around said rotation axis (21) of the hinge device (19), said shell (23) also comprising a pair of slides (28) superimposed and coplanar with said pair of disks (24), wherein each of said slides (28A, 28B) has a fitting element (29) at one end for said perimetric teeth (25) of said disks (24A, 24B), and wherein said slides (28A, 28B) are each coupled with regulation means (35A, 35B, 36A, 36B) for engagement with or disengagement from the disks (24A, 24B).

2. The rod according to claim 1, **characterized in that** said disks (24A, 24B) each comprise a number of teeth (25) equal to the number of discreet angular positions between said rod portions (15, 16).

3. The rod according to claim 1, **characterized in that** said disks (24A, 24B) are made integral with each other by means of a peg (26) inserted in aligned holes.

4. The rod according to claim 1, **characterized in that** said regulation means, suitable for causing a translation of each of said slides (28A, 28B) along a translation axis (30) orthogonal to said rotation axis (21), comprise two toothed wheels (35A, 35B), each of said wheels (35A, 35B) being coplanar with one of said slides (28A, 28B), wherein said slides (28A, 28B) have at least on one side, parallel to said translation axis (30), a first toothing (34), or a series of crests and cavities, to be coupled with said toothed wheels (35A, 35B).

5. The rod according to claim 4, **characterized in that** said wheels (35A, 35B) are applied to a fixed pin (38) and are partially protruding towards the outside of said shell (23) for activation on the part of the user.

6. The rod according to claim 5, **characterized in that** said wheels (35A, 35B) are respectively situated on opposite sides with respect to said pair of slides (28).

7. The rod according to claim 4, **characterized in that** said regulation means comprise at least one cursor (36A, 36B) comprising a toothed portion (37) complementary to said at least one toothed side of said slides (28) as well as at least one elastic element (41) suitable for keeping said cursor (36A, 36B) engaged with said slides (28A, 28B).

8. The rod according to claim 7, **characterized in that** said regulation means comprise two cursors (36A, 36B) respectively engaged with said two slides (28A, 28B) .

9. The rod according to claim 8, **characterized in that** each of said slides (28A, 28B) has a second toothing (34') on the opposite side to said first toothing (34), each of said slides (28A, 28B) being respectively engaged on opposite sides with a regulation wheel (35A, 35B), with a toothing complementary to said first toothing (34), and with said cursor (36A, 36B) having a toothed portion (37) complementary to said second toothing (34').

10. The rod according to claim 9, **characterized in that** said second toothing (34') has a greater pitch with respect to a pitch of said first toothing (34), said pitch corresponding to an angular pitch.

11. The rod according to claim 7, **characterized in that** said at least one cursor (36A, 36B) is made of antifriction material.

12. The rod according to claim 7, **characterized in that** said at least one cursor (36A, 36B) and said at least one elastic element (41) are housed in said shell (23) in at least one housing (40).

13. The rod according to claim 1, **characterized in that** said shell (23) comprises at least one peg (31) suitable for guiding the translation of said pair of slides (28).

14. The rod according to claim 1, **characterized in that** said shell (23) is equipped with at least one window (42), or opening, and a graded scale (43) for the reading of the angular position established between said two rod portions (15, 16).

15. The rod according to claim 14, **characterized in that** said slides (28) comprise a moveable indicator (44) in said window (42).

16. The rod according to claim 1, **characterized in that** it comprises a stable blocking screw (33) of the angular position between said two rod portions (15, 16).

17. The rod according to claim 16, **characterized in that** said screw (33) is inserted in said pair of slides (28) in a central slit (32) parallel to said translation axis (30).

18. The rod according to claim 5, **characterized in that** on said fixed pin (38), there is also a ring (39) made of antifriction plastic material in contact with said regulation wheel (35A, 35B), said antifriction ring (39) and said regulation wheel (35A, 35B) each being coplanar with one of said superimposed slides (28).

19. The rod according to claim 1, **characterized in that** it comprises at least one further articulated joint (20) situated between said hinge device (19) and one of said elements (15, 16) of the brace suitable for allowing a rotational movement around a second rotation axis (22), said rotation axis (22) of the articulated joint (20) and said rotation axis (21) of the hinge device (19) being orthogonal with each other.

20. The articulated rod according to claim 19, **characterized in that** said articulated joint (20) comprises disk portions (45) which can be rotated around said second rotation axis (22), said disk portions (45) each being equipped with flat facing surfaces equipped with friction means (47).

21. The rod according to claim 20, **characterized in that** said disk portions (45) have a central pass-through hole for the insertion of a regulation screw.

22. The rod according to claim 20, **characterized in that** said friction means (47) are complementary toothings.

## Patentansprüche

1. Gelenkstange mit unterstützter Einstellung für eine orthopädische Stütze, umfassend zwei Stangenabschnitte (15, 16), welche durch wenigstens eine Gelenkvorrichtung (19) für eine Drehbewegung um eine erste Drehachse (21) der Stütze verbunden sind, wobei die Stangenabschnitte (15, 16) fest mit einem ersten und einem zweiten Element einer orthopädischen Stütze (12, 13) verbunden sind, **dadurch gekennzeichnet, dass** die Gelenkvorrichtung (19) eine Schale (23) und ein Paar von fest an den Stangenabschnitten (15, 16) angebrachten Scheiben (24) umfasst, wobei jede der Scheiben (24A, 24B) einen Umfang mit einem zunehmenden Durchmesser aufweist, welcher mit einer Reihe von Zähnen (25) oder Stufen ausgestattet ist, wobei die Scheiben (24) in der Schale (23) mit gegenüberliegenden Zahnprofilen (25) überlagert sind und integral zusammen um die Drehachse (21) der Gelenkvorrichtung (19) gedreht werden können, wobei die Schale (23) auch ein Paar von Schiebern (28) umfasst, welche überlagert und koplanar zu dem Paar von Scheiben (24) sind, wobei jeder der Schieber (28A, 28B) an einem Ende ein Passelement (29) für die Umfangszähne (25) der Scheiben (24A, 24B) aufweist und wobei die Schieber (28A, 28B) jeweils mit Einstellmitteln (35A, 35B, 36A, 36B) zum Eingriff mit oder Lösen von den Scheiben (24A, 24B) gekoppelt sind.

2. Stange nach Anspruch 1, **dadurch gekennzeichnet, dass** die Scheiben (24A, 24B) jeweils eine Anzahl von Zähnen (25) umfassen, welche gleich der Anzahl von unterschiedlichen Winkelpositionen zwischen den Stangenabschnitten (15, 16) ist.

3. Stange nach Anspruch 1, **dadurch gekennzeichnet, dass** die Scheiben (24A, 24B) mittels eines in ausgerichtete Löcher eingesetzten Stifts (26) miteinander integral gemacht sind.

4. Stange nach Anspruch 1, **dadurch gekennzeichnet, dass** die Einstellmittel, welche zum Bewirken einer Verschiebung von jedem der Schieber (28A, 28B) entlang einer zu der Drehachse (21) senkrechten Verschiebungsachse (30) geeignet sind, zwei gezahnte Räder (35A, 35B) umfassen, wobei jedes der Räder (35A, 35B) koplanar zu einem der Schieber (28A, 28B) ist, wobei die Schieber (28A, 28B) wenigstens auf einer Seite parallel zu der Verschiebungsachse (30) eine erste Zahnung oder eine Reihe von Spitzen und Vertiefungen aufweisen, um mit den gezahnten Rädern (35A, 35B) gekoppelt zu sein.

5. Stange nach Anspruch 4, **dadurch gekennzeichnet, dass** die Räder (35A, 35B) an einem feststehenden Stift (38) angebracht sind und zur Aktivierung seitens des Benutzers teilweise in Richtung der Außenseite der Schale (23) hervorstehen.

6. Stange nach Anspruch 5, **dadurch gekennzeichnet, dass** die Räder (35A, 35B) sich jeweils auf gegenüberliegenden Seiten bezüglich des Paars von Schiebern (28) befinden.

7. Stange nach Anspruch 4, **dadurch gekennzeichnet, dass** die Einstellmittel wenigstens einen Positionsanzeiger (36A, 36B) umfassen, welcher einen zu der wenigstens einen gezahnten Seite der Schieber (28) komplementären gezahnten Abschnitt (37) sowie wenigstens ein elastisches Element (41), welches zum Halten des Positionsanzeigers (36A, 36B) im Eingriff mit den Schiebern (28A, 28B) geeignet ist, umfasst.

8. Stange nach Anspruch 7, **dadurch gekennzeichnet, dass** die Einstellmittel zwei Positionsanzeiger (36A, 36B) umfassen, welche entsprechend mit den zwei Schiebern (28A, 28B) im Eingriff sind.

9. Stange nach Anspruch 8, **dadurch gekennzeichnet, dass** jeder der Schieber (28A, 28B) eine zweite Zahnung (34') auf der zu der ersten Zahnung (34) gegenüberliegenden Seite aufweist, wobei jeder der Schieber (28A, 28B) jeweils auf gegenüberliegenden Seiten mit einem Einstellrad (35A, 35B) mit einer zu der ersten Zahnung (34) komplementären Zahnung und mit dem Positionsanzeiger (36A, 36B), welcher einen zu der zweiten Zahnung (34') komplementären gezahnten Abschnitt (37) aufweist, im Eingriff ist.

10. Stange nach Anspruch 9, **dadurch gekennzeichnet, dass** bezüglich einer Schrittweite der ersten Zahnung (34) die zweite Zahnung (34') eine größere Schrittweite aufweist, wobei die Schrittweite einer Winkelschrittweite entspricht.

11. Stange nach Anspruch 7, **dadurch gekennzeichnet, dass** der wenigstens eine Positionsanzeiger (36A, 36B) aus einem Gleitmaterial gefertigt ist.

12. Stange nach Anspruch 7, **dadurch gekennzeichnet, dass** der wenigstens eine Positionsanzeiger (36A, 36B) und das wenigstens eine elastische Element (41) in der Schale (23) in wenigstens einem Gehäuse (40) untergebracht sind.

13. Stange nach Anspruch 1, **dadurch gekennzeichnet, dass** die Schale (23) wenigstens einen Stift (31) umfasst, welcher dazu geeignet ist, die Verschiebung des Paars von Schiebern (28) zu führen.

14. Stange nach Anspruch 1, **dadurch gekennzeichnet, dass** die Schale (23) mit wenigstens einem Fenster (42) oder wenigstens einer Öffnung und einer Stufenskala (43) zum Ablesen der zwischen den zwei Stangenabschnitten (15, 16) hergestellten Winkelposition ausgestattet ist.

15. Stange nach Anspruch 14, **dadurch gekennzeichnet, dass** die Schieber (28) ein bewegliches Anzeigeelement (44) in dem Fenster (42) umfassen.

16. Stange nach Anspruch 1, **dadurch gekennzeichnet, dass** sie eine Schraube (33) zum stabilen Feststellen der Winkelposition zwischen den zwei Stangenabschnitten (15, 16) umfasst.

17. Stange nach Anspruch 16, **dadurch gekennzeichnet, dass** die Schraube (33) in einem zu der Verschiebungsachse (30) parallelen Mittelschlitz (32) in das Paar von Schiebern (28) eingesetzt ist.

18. Stange nach Anspruch 5, **dadurch gekennzeichnet, dass** an dem feststehenden Stift (38) auch ein Ring (39) vorhanden ist, welcher aus einem Kunststoffgleitmaterial gefertigt ist und in Kontakt mit dem Einstellrad (35A, 35B) ist, wobei der Gleitring (39) und das Einstellrad (35A, 35B) jeweils koplanar mit einem der überlagerten Schieber (28) ist.

19. Stange nach Anspruch 1, **dadurch gekennzeichnet, dass** sie wenigstens eine weitere Gelenkverbindung (20) umfasst, welche sich zwischen der Gelenkvorrichtung (19) und einem der Elemente (15, 16) der Stütze befindet und dazu geeignet ist, eine Drehbewegung um eine zweite Drehachse (22) zu ermöglichen, wobei die Drehachse (22) der Gelenkverbindung (20) und die Drehachse (21) der Gelenkvorrichtung (19) zueinander senkrecht sind.

20. Gelenkstange nach Anspruch 19, **dadurch gekennzeichnet, dass** die Gelenkverbindung (20) Scheibenabschnitte (45) umfasst, welche um die zweite Drehachse (22) gedreht werden können, wobei die Scheibenabschnitte (45) jeweils mit flachen Stirnflächen ausgestattet sind, die mit Reibungsmitteln (47) ausgestattet sind.

21. Stange nach Anspruch 20, **dadurch gekennzeichnet, dass** die Scheibenabschnitte (45) ein zentrales Durchgangsloch zum Einsetzen einer Einstellschraube aufweisen.

22. Stange nach Anspruch 20, **dadurch gekennzeichnet, dass** die Reibungsmittel (47) komplementäre Zahnungen sind.

## Revendications

1. Tige articulée avec réglage assisté pour un appareil orthopédique comprenant deux parties de tige (15, 16) reliées par au moins un dispositif d'articulation (19) pour un mouvement de rotation autour d'un premier axe de rotation (21) de l'appareil, lesdites parties de tige (15, 16) étant solidement raccordées à un premier et un deuxième élément d'un appareil orthopédique (12, 13), **caractérisée en ce que** ledit dispositif d'articulation (19) comprend une coque (23) et une paire de disques (24) solidement appliquée sur lesdites parties de tige (15, 16), dans laquelle chacun des disques (24A, 24B) présente un périmètre avec un diamètre croissant muni d'une série de dents (25) ou de gradins, lesdits disques (24) étant superposés dans ladite coque (23) avec des profils dentés opposés (25), et qui peuvent tourner solidairement ensemble autour dudit axe de rotation (21) du dispositif d'articulation (19), ladite coque (23) comprenant également une paire de glissières (28) superposées et situées dans le même plan que ladite paire de disques (24), dans laquelle chacune desdites glissières (28A, 28B) comporte un élément d'ajustement (29) à une extrémité pour lesdites dents périmétrales (25) desdites, glissières (28A, 28B), et dans laquelle lesdites glissières (28A, 28B) sont couplées chacune à des moyens de réglage (35A, 35B, 36A, 36B) pour s'engager dans les ou se désengager des disques (24A, 24B).

2. Tige selon la revendication 1, **caractérisée en ce que** lesdits disques (24A, 24B) comprennent chacun un nombre de dents (25) égal au nombre de positions angulaires discrètes entre lesdites parties de tige (15, 16).

3. Tige selon la revendication 1, **caractérisée en ce que** lesdits disques (24A, 24B) sont rendus solidaires les uns des autres à l'aide d'une cheville (26) insérée dans des trous alignés.

4. Tige selon la revendication 1, **caractérisée en ce que** lesdits moyens de réglage, appropriés pour produire une translation de chacune desdites glissières (28A, 28B) le long d'un axe de translation (30) orthogonal audit axe de rotation (21), comprennent deux roues dentées (35A, 35B), chacune desdites roues (35A, 35B) étant située dans le même plan que l'une desdites glissières (28A, 28B), dans laquelle lesdites glissières (28A, 28B) ont au moins un côté, parallèle audit axe de translation (30), une première denture (34), ou une série de crêtes et de cavités à coupler avec lesdites roues dentées (35A, 358).

5. Tige selon la revendication 4, **caractérisée en ce que** lesdites roues (35A, 35B) sont appliquées sur une goupille fixe (38) et font partiellement saillie vers l'extérieur de ladite coque (23) pour l'activation de la part de l'utilisateur.

6. Tige selon la revendication 5, **caractérisée en ce que** lesdites roues (35A, 35B) sont respectivement situées sur des côtés opposés par rapport à ladite paire de glissières (28).

7. Tige selon la revendication 4, **caractérisée en ce que** lesdits moyens de réglage comprennent au moins un curseur (36A, 36B) comprenant une partie dentée (37) complémentaire dudit au moins un côté denté desdites glissières (28) ainsi qu'au moins un élément élastique (41) approprié pour maintenir ledit curseur (36A, 36B) engagé dans lesdites glissières (28A, 28B),

8. Tige selon la revendication 7, **caractérisée en ce que** lesdits moyens de réglage comprennent deux curseurs (36A, 36B) respectivement engagés dans lesdites deux glissières (28A, 28B).

9. Tige selon la revendication 8, **caractérisée en ce que** chacune desdites glissières (28A, 28B) comporte une deuxième denture (34') sur le côté opposé de ladite première denture (34), chacune desdites glissières (28A, 28B) étant respectivement engagée sur des côtés opposés dans une roue de réglage (35A, 35B), avec une denture complémentaire de ladite première denture (34), et ledit curseur (36A, 36B) ayant une partie dentée (37) complémentaire de ladite deuxième denture (34').

10. Tige selon la revendication 9, **caractérisée en ce que** ladite deuxième denture (34') a un pas supérieur à un pas de ladite première denture (34), ledit pas correspondant à un pas angulaire.

11. Tige selon la revendication 7, **caractérisée en ce que** ledit au moins un curseur (36A, 36B) est constitué d'une matière antifriction.

12. Tige selon la revendication 7, **caractérisée en ce que** ledit au moins un curseur (36A, 36B) et ledit au moins un élément élastique (41) sont logés dans ladite coque (23) dans au moins un logement (40).

13. Tige selon la revendication 1, **caractérisée en ce que** ladite coque (23) comprend au moins une cheville (26) appropriée pour guider la translation de ladite paire de glissières (28).

14. Tige selon la revendication 1, **caractérisée en ce que** ladite coque (23) est équipée d'au moins une fenêtre (42), ou ouverture, et d'une échelle graduée (43) pour la lecture de la position angulaire établie entre lesdites deux parties de tige (15, 16).

15. Tige selon la revendication 14, **caractérisée en ce que** lesdites glissières (28) comprennent un indicateur mobile (44) dans ladite fenêtre (42).

16. Tige selon la revendication 1, **caractérisée en ce qu'**elle comprend une vis de blocage stable (33) de la position angulaire entre lesdites deux parties de tige (15, 16) .

17. Tige selon la revendication 16, **caractérisée en ce que** ladite vis (33) est insérée dans ladite paire de glissières (28) dans une fente centrale (32) parallèle audit axe de translation (30).

18. Tige selon la revendication 5, **caractérisée en ce que**, sur ladite goupille fixe (38), il y a également une bague (39) constituée d'une matière plastique antifriction en contact avec ladite roue de réglage (35A, 35B), ladite bague antifriction (39) et ladite roue de réglage (35A, 35B) étant situées chacune dans le même plan que l'une desdites glissières superposées (28).

19. Tige articulée selon la revendication 1, **caractérisée en ce qu'**elle comprend au moins un autre joint articulé (20) situé entre ledit dispositif d'articulation (19) et l'un desdits éléments (15, 16) de l'appareil approprié pour permettre un mouvement de rotation autour d'un deuxième axe de rotation (22), ledit axe de rotation (22) du joint articulé (20) et ledit axe de rotation (21) du dispositif d'articulation (19) étant orthogonaux l'un par rapport à l'autre.

20. Tige articulée selon la revendication 19, **caractérisée en ce que** ledit joint articulé (20) comprend des parties de disque (45) qui peuvent tourner autour dudit axe de rotation (22), chacune desdites parties de disque (45) étant équipée de surfaces de parement plates équipées de moyens de friction (47),

21. Tige articulée selon la revendication 20, **caractérisée en ce que** lesdites parties de disque (45) comportent un trou passant central pour l'insertion d'une vis de réglage,

22. Tige articulée selon la revendication 20, **caractérisée en ce que** lesdits moyens de friction (47) sont des dentures complémentaires.
